# EUROPÄISCHE PATENTSCHRIFT

(11) **EP 1 828 142 B1**
(45) Veröffentlichungstag und Bekanntmachung des Hinweises auf die Patenterteilung: **02.06.2010**
(21) Anmeldenummer: 05808712.3
(22) Anmeldetag: 18.11.2005
(51) Int. Cl.: C07D 233/58, C07F 9/54, C07D 213/18, C07D 295/037, C07C 279/02, C07C 275/02

(54) **VERFAHREN ZUR HERSTELLUNG VON ONIUM ALKYLSULFATEN MIT GERINGEM HALOGENID-GEHALT**
METHOD FOR PRODUCING ONIUM ALKYL SULFATES WITH A LOW HALOGENIDE CONTENT
PROCEDE POUR PRODUIRE DES SULFATES D'ALKYLE D'ONIUM A FAIBLE TENEUR EN HALOGENURE

(30) Priorität: 14.12.2004 DE 102004060074
(43) Veröffentlichungstag der Anmeldung: 05.09.2007
(73) Patentinhaber: Merck Patent GmbH, 64293 Darmstadt (DE)
(72) Erfinder: IGNATYEV, Nicolai, 47058 Duisburg (DE); WELZ-BIERMANN, Urs, 64646 Heppenheim (DE); KUCHERYNA, Andriy, 42117 Wuppertal (DE); WILLNER, Helge, 45481 Muelheim/Ruhr (DE)
(86) Internationale Anmeldenummer: PCT/EP2005/012399
(87) Internationale Veröffentlichungsnummer: WO 2006/063654

(56) Entgegenhaltungen:
- DE-A1- 10 319 465
- US-A- 2 585 979
- PETER WASSERSCHEID ET AL.: GREEN CHEMISTRY, Bd. 4, 2002, Seiten 400-404, XP008024341

## Beschreibung

Die Erfindung betrifft ein Verfahren zur Herstellung von Onium Alkylsulfaten durch Umsetzung eines Onium Halogenids mit einem symmetrisch substituierten Dialkylsulfat, wobei die Alkylgruppe 1 bis 14 C-Atome haben kann, mit einem unsymmetrisch substituierten Dialkylsulfat, wobei eine Alkylgruppe 4 bis 20 C-Atome haben kann und die zweite Alkylgruppe Methyl oder Ethyl bedeutet, mit einem Alkyl-trialkylsilyl-sulfat, mit einem Alkyl-acyl-sulfat oder mit einem Alkyl-sulfonyl-sulfat, wobei die Umsetzung mit einem Dialkylsulfat bei Raumtemperatur stattfindet.

Eine große Anzahl von Onium-Salzen, insbesondere Alkylsulfate, sind ionische Flüssigkeiten. Ionische Flüssigkeiten stellen durch Ihre Eigenschaften in der modernen Forschung eine wirksame Alternative zu traditionellen flüchtigen organischen Solventien für die organische Synthese dar. Die Verwendung von ionischen Flüssigkeiten als neues Reaktionsmedium könnte weiterhin eine praktische Lösung sowohl für die Lösungsmittel Emission als auch für Probleme in der Wiederaufbereitung von Katalysatoren sein.

Ionische Flüssigkeiten oder flüssige Salze sind ionische Spezies, die aus einem organischen Kation und einem in der Regel anorganischen Anion bestehen. Sie enthalten keine neutralen Moleküle und weisen meistens Schmelzpunkte kleiner 373 K auf. Der Schmelzpunkt kann jedoch auch höher liegen, ohne die Anwendbarkeit der Salze auf allen Anwendungsgebieten zu beschränken. Beispiele für organische Kationen sind unter anderem Tetraalkylammonium, Tetraalkylphosphonium, N-Alkylpyridinium, 1,3-Dialkylimidazolium oder Trialkylsulfonium. Unter einer Vielzahl von geeigneten Anionen sind beispielsweise BF₄⁻, PF₆⁻, SbF₆⁻, NO₃⁻, CF₃SO₃⁻, (CF₃SO₂)₂N⁻, ArylSO₃⁻, CF₃CO₂⁻, CH₃CO₂⁻ oder Al₂Cl₇⁻ zu nennen.

Eine generelle Methode zur Herstellung von Onium-Alkylsulfaten ist die Alkylierung der organischen Base, d.h. beispielsweise des Amins, Phosphins, Guanidins oder der heterocyclischen Base, mit Dialkylsulfaten, auch bekannt durch die Veröffentlichung von John D. Holbrey et al, Green Chemistry (2002), 4(5), 407-413. Ein Nachteil dieser Methode ist jedoch, dass ein Substituent des entstehenden Onium Alkylsulfats immer der entsprechenden Alkylgruppe des Dialkylsulfats entspricht. Zur Herstellung von Onium Alkylsulfaten, deren Alkylgruppe im Anion von den Substituenten des Kations unterschiedlich ist, im folgenden als unsymmetrisch substituierte Onium Alkylsulfate bezeichnet, müsste man unsymmetrisch substituierte Dialkylsulfate einsetzen, beispielsweise Ethylmethylsulfat, welche zu gemischt alkylierten Onium Alkylsulfaten führen. Zum einen würde die organische Base ethyliert und man erhielte ein Methylsulfat, zum anderen würde die organische Base methyliert und man erhielte ein Ethylsulfat.

Unsymmetrische Onium Alkylsulfate, wie zuvor definiert, z.B. 1-Butyl-3-methylimidazolium Octylsulfat, können nach P. Wasserscheid et al, Green Chemistry (2002), 4(4), 400-404 auch durch Umsetzung des Oniumhalogenids, z.B. 1-Butyl-3-methylimidazoliumchlorid, mit einem entsprechenden Alkalimetallsulfat, z.B. Natriumoctylsulfat, synthetisiert werden, wobei jedoch das anfallende Alkalimetallhalogenid, z.B. Natriumchlorid, durch eine zusätzliche Aufreinigungsmethode entfernt werden muss. Die Verunreinigung durch Halogenid-lonen, beispielsweise Chlorid-lonen, größer als 1000 ppm (0,1 %), reduziert die Anwendbarkeit der ionischen Flüssigkeit, insbesondere in der Anwendung für elektrochemische Prozesse. Daher ist bei Verfahren zur Herstellung von Onium-Salzen, insbesondere ionischer Flüssigkeiten, die Technologie von entscheidender Bedeutung, damit diese durch die Reaktion per se oder die Reaktionsführung mit geringen Verunreinigungen synthetisiert werden können und so weitere kostenintensive zusätzliche Verfahrensschritte bei der Synthese entfallen.

Aufgabe der vorliegenden Erfindung war dementsprechend ein alternatives Verfahren zur Herstellung von Onium Alkylsulfaten mit geringem Halogenid-Gehalt zur Verfügung zu stellen, welches zu Alkylsulfaten, vorzugsweise von unsymmetrisch substituierten Onium Alkylsulfaten, mit hoher Reinheit in guter Ausbeute führt und auch für eine großtechnische Produktion geeignet ist.

Selbstverständlich ist ein solches Verfahren dann auch zur Herstellung von symmetrisch substituierten Onium Alkylsulfaten geeignet. Die erfindungsgemäße Methode ist auch für die Reinigung von halogenidhaltigen Onium Alkylsulfaten verwendbar.

Die Aufgabe wird durch das erfindungsgemäße Verfahren gelöst, da das symmetrisch substituierte Dialkylsulfat, wobei die Alkylgruppen 1 bis 14 C-Atome haben kann, das unsymmetrisch substituierte Dialkylsulfat, wobei eine Alkylgruppe 4 bis 20 C-Atome haben kann und die zweite Alkylgruppe Methyl oder Ethyl bedeutet, das Alkyl-trialkylsilyl-sulfat, das Alkyl-acyl-sulfat oder Alkyl-sulfonyl-sulfat das Anion des eingesetzten Oniumhalogenids alkyliert und nicht das organische Kation. Die als Nebenprodukt entstehenden Alkyl-, Acyl-, Trialkylsilyl- oder Sulfonylhalogenide sind in der Regel Gase oder leicht flüchtige Verbindungen, die ohne großen prozesstechnischen Aufwand aus der Reaktionsmischung entfernt werden können.

Gegenstand der Erfindung ist daher ein Verfahren zur Herstellung von Onium Alkylsulfaten, insbesondere von unsymmetrisch substituierten Onium Alkylsulfaten, durch Umsetzung eines Onium Halogenids mit einem symmetrisch substituierten Dialkylsulfat, wobei die Alkylgruppe 1 bis 14 C-Atome haben kann, mit einem unsymmetrisch substituierten Dialkylsulfat,
wobei eine Alkylgruppe 4 bis 20 C-Atome haben kann und die zweite Alkylgruppe Methyl oder Ethyl bedeutet, mit einem Alkyl-trialkylsilyl-sulfat, mit einem Alkyl-acyl-sulfat oder mit einem Alkyl-sulfonyl-sulfat, wobei die Umsetzung mit einem Dialkylsulfat bei Raumtemperatur stattfindet.

Bei Verwendung eines C₄-C₂₀-Alkyl-methyl- oder C₄-C₂₀-Alkyl-ethyl-sulfats vermeidet man die Entstehung von Gemischen, da die Methylgruppe oder Ethylgruppe reaktiver ist und das Halogenid methylieren bzw. ethylieren wird und sich das Alkylsulfat mit 4 bis 20 C-Atomen das Anion des Onium Alkylsulfats am meisten bildet.

Selbstverständlich ist der Einsatz sowohl von symmetrisch substituierten Dialkylsulfaten mit 1 bis 20 C-Atomen oder der Einsatz von unsymmetrisch substituierten Dialkylsulfaten mit 1 bis 20 C-Atomen oder noch höher alkylierten Edukten im erfindungsgemäßen Verfahren möglich. Der Vorteil liegt immer darin, dass die entstehenden Onium Alkylsulfate halogenidreduziert hergestellt worden sind.

Aus US 2,585,979 ist die Herstellung von quaternären Sulfaten von Pyrimidyl-aminochinolin-Derivaten 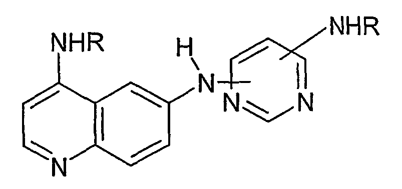 durch Umsetzung des entsprechenden Pyrimidyl-aminochinolin Halogenids mit Dimethyl- oder Diethylsulfat bekannt. Die Reaktion findet jedoch im Gegensatz zum erfindungsgemäßen Verfahren bei Temperaturen von 90° bis 150°C in Gegenwart eines Lösungsmittels, beispielsweise Nitrobenzen, statt. Überraschenderweise gelingt jedoch die erfindungsgemäße Umsetzung bei Raumtemperatur, d.h. bei Temperaturen zwischen 10° und 30°C, ohne Einsatz eines Lösungsmittels in annähernd quantitativer Ausbeute.

Die gleiche technische Lehre der US 2,585,979 wird auch in Vompe et al, J. Org. Chem. USSR (engl. Transl), 17, 1981, 1551-1554 vermittelt. Dort wird offenbart, dass bei der Reaktion von 2-Methyl-3-ethylnaphtho[2,1-d]thiazoliumiodid mit Dimethylsulfat bei 80°-130°C ein Gemisch von Methylsulfat und das Bisulfat (Hydrogensulfat) entsteht, wohingehend bei Temperaturen von 130°C das Bisulfat HSO₄⁻ erhalten wird. Auch hier werden hohe Temperaturen gefordert.

Das erfindungsgemäße Verfahren bei Verwendung der insbesondere symmetrisch substituierten Dialkylsulfate als Reagenz ist daher als Auswahlerfindung aus den Verfahren des Standes der Technik zu sehen. Ein Hinweis auf die Verwendung der Reagenzien Alkyl-trialkylsilyl-sulfat, Alkyl-acyl-sulfat oder Alkyl-sulfonyl-sulfat findet sich nicht.

Geeignete Onium Halogenide sind Ammoniumhalogenide, Phosphoniumhalogenide, Thiouroniumhalogenide, Guanidiniumhalogenide oder Halogenide mit heterocyclischem Kation, wobei die Halogenide aus der Gruppe Chloride oder Bromide ausgewählt werden können. Bevorzugt werden im erfindungsgemäßen Verfahren Phosphonium-, Thiouronium-, Guanidiniumhalogenide oder Halogenide mit heterocyclischem Kation eingesetzt. Neben den Chloriden und Bromiden sind die Thiouroniumiodide besonders geeignet.

Die Onium Halogenide sind in der Regel kommerziell erhältlich oder können nach Syntheseverfahren hergestellt werden, wie sie aus der Literatur, z.B. in den Standardwerken wie Houben-Weyl, Methoden der organischen Chemie, Georg-Thieme-Verlag, Stuttgart oder Richard C. Larock, Comprehensive Organic Transformations, 2nd Edition, Wiley-VCH, New York, 1999 bekannt sind. Dabei kann man auch von an sich bekannten, hier nicht näher erwähnten Varianten Gebrauch machen.

Phosphoniumhalogenide können beispielsweise durch die Formel (1) beschrieben werden,

[PR₄]⁺ Hal⁻ (1),

wobei
Hal Cl oder Br und
R jeweils unabhängig voneinander
H, wobei nicht alle Substituenten R gleichzeitig H sein dürfen, geradkettiges oder verzweigtes Alkyl mit 1-20 C-Atomen,
geradkettiges oder verzweigtes Alkenyl mit 2-20 C-Atomen und einer oder mehreren Doppelbindungen bedeutet,
wobei ein oder mehrere R teilweise oder vollständig mit Halogenen, insbesondere -F und/oder -Cl, oder teilweise mit -NO₂ substituiert sein können, wobei jedoch nicht alle vier oder drei R vollständig mit Halogenen substituiert sein dürfen.

Ausgeschlossen sind demnach Verbindungen der Formel (1), in denen alle vier oder drei Substituenten R vollständig mit Halogenen substituiert sind, beispielsweise Tris(trifluormethyl)methylphosphoniumchlorid, Tetra(trifluormethyl)phosphoniumchlorid oder Tetra(nonafluorbutyl)phosphoniumchlorid.

Guanidiniumhalogenide können beispielsweise durch die Formel (2) beschrieben werden,

[C(NR¹R²)(NR³R⁴)(NR⁵R⁶)⁺ Hal⁻ (2),

wobei
Hal Cl oder Br und
R¹ bis R⁶ jeweils unabhängig voneinander
Wasserstoff,
geradkettiges oder verzweigtes Alkyl mit 1 bis 20 C-Atomen,
geradkettiges oder verzweigtes Alkenyl mit 2-20 C-Atomen und einer oder mehreren Doppelbindungen, bedeutet,
wobei ein oder mehrere der Substituenten R¹ bis R⁶ teilweise oder vollständig mit Halogenen, insbesondere -F und/oder -Cl, oder teilweise mit -NO₂, substituiert sein können, wobei jedoch nicht alle Substituenten an einem N-Atom vollständig mit Halogenen substituiert sein dürfen und
wobei die Substituenten R¹ bis R⁶ paarweise durch Einfach- oder Doppelbindung miteinander verbunden sein können

Thiouroniumhalogenide können beispielsweise durch die Formel (3)

[(R¹R²N)-C(=SR⁷)(NR³R⁴)]⁺ Hal⁻ (3

beschrieben werden,
wobei
Hal Cl, Br oder I und
R¹ bis R⁷ jeweils unabhängig voneinander
Wasserstoff, wobei Wasserstoff für R⁷ ausgeschlossen wird,
geradkettiges oder verzweigtes Alkyl mit 1 bis 20 C-Atomen,
geradkettiges oder verzweigtes Alkenyl mit 2-20 C-Atomen und einer oder mehreren Doppelbindungen, bedeutet,
wobei ein oder mehrere der Substituenten R¹ bis R⁷ teilsweise oder vollständig mit Halogenen, insbesondere -F und/oder -Cl, oder teilweise mit -NO₂, substituiert sein können, wobei jedoch nicht alle Substituenten an einem N-Atom vollständig mit Halogenen substituiert sein dürfen und
wobei die Substituenten R¹ bis R⁷ paarweise durch Einfach- oder Doppelbindung miteinander verbunden sein können.

Halogenide mit heterocyclischem Kation können beispielsweise durch die Formel (4) beschrieben werden,

[HetN]⁺ Hal⁻ (4),

wobei
Hal Cl oder Br und
HetN⁺ ein heterocyclisches Kation, ausgewählt aus der Gruppe 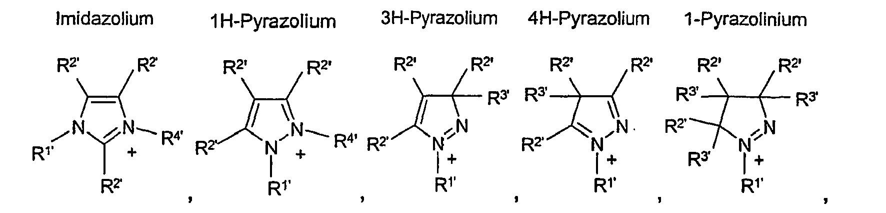 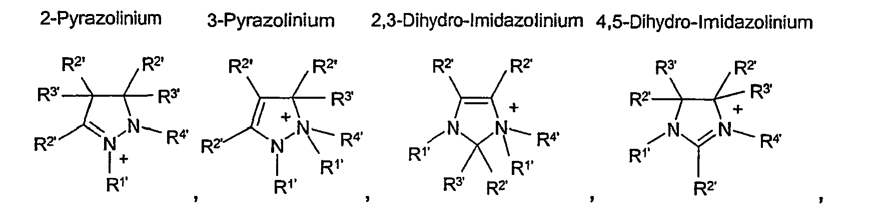 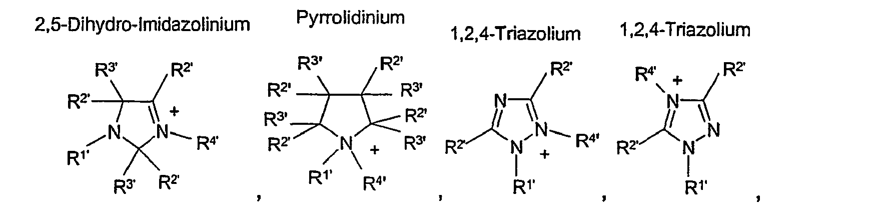 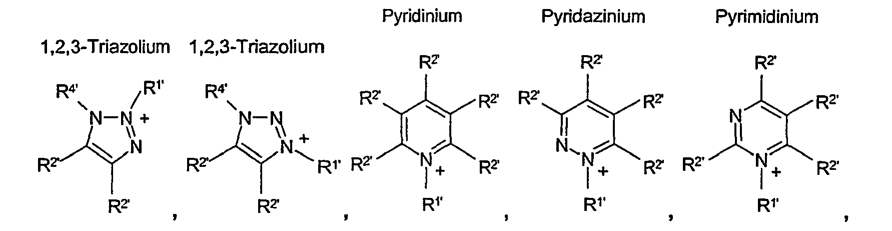 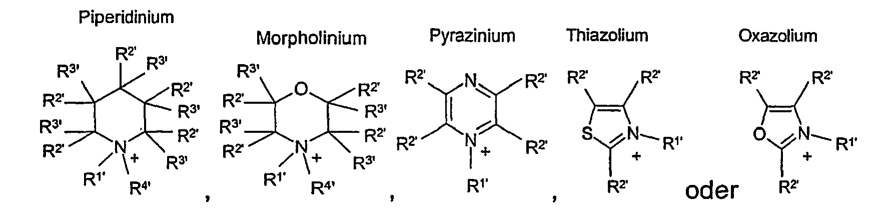 bedeutet, wobei die Substituenten
R¹' bis R⁴' jeweils unabhängig voneinander
Wasserstoff oder CN,
geradkettiges oder verzweigtes Alkyl mit 1-20 C-Atomen,
geradkettiges oder verzweigtes Alkenyl mit 2-20 C-Atomen und einer oder mehreren Doppelbindungen oder
Aryl-C₁-C₆-alkyl bedeuten,
wobei ein oder mehrere Substituenten R¹' bis R⁴' teilweise oder vollständig mit Halogenen, insbesondere -F und/oder -Cl, oder teilweise mit -NO₂ oder CN substituiert sein können, wobei jedoch nicht gleichzeitig R^{1'} und R^{4'} vollständig mit Halogenen substituiert sein dürfen, und für R¹' bis R⁴' = Aryl-C₁-C₆-alkyl, sowohl der Phenylring als auch die Alkylenkette teilweise oder vollständig mit Halogenen oder teilweise mit -NO₂ substituiert sein können.

Die C₁-C₁₄-Alkylgruppe ist beispielsweise Methyl, Ethyl, Isopropyl, Propyl, Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, Heptyl, Octyl, Nonyl, Decyl, Undecyl, Dodecyl, Tridecyl oder Tetradecyl.

Ein geradkettiges oder verzweigtes Alkenyl mit 2 bis 20 C-Atomen, wobei auch mehrere Doppelbindungen vorhanden sein können, ist beispielsweise Vinyl, Allyl, 2- oder 3-Butenyl, Isobutenyl, sek.-Butenyl, ferner 4-Pentenyl, iso-Pentenyl, Hexenyl, Heptenyl, Octenyl, -C₉H₁₇, -C₁₀H₁₉ bis -C₂₀H₃₉; vorzugsweise Vinyl, Allyl, 2- oder 3-Butenyl, isobutenyl, sek.-Butenyl, ferner bevorzugt ist 4-Pentenyl, iso-Pentenyl oder Hexenyl.

Aryl-C₁-C₆-alkyl bedeutet beispielsweise Benzyl, Phenylethyl, Phenylpropyl, Phenylbutyl, Phenylpentyl oder Phenylhexyl, wobei sowohl der Phenylring als auch die Alkylenkette, wie zuvor beschrieben teilweise oder vollständig mit Halogenen, insbesondere -F und/oder -Cl, oder teilsweise mit -NO₂, substituiert sein können, besonders bevorzugt Benzyl oder Phenylpropyl.

Als Substituenten R und R¹ bis R⁷ der Verbindungen der Formeln (1) bis (3) kommen erfindungsgemäß dabei jeweils unabhängig voneinander neben Wasserstoff, wobei Wasserstoff für R⁷ ausgeschlossen wird, bevorzugt in Frage: C₁- bis C₂₀-, insbesondere C₁- bis C₁₄-Alkylgruppen.
Die Substituenten R und R¹ bis R⁷ können jedoch ebenfalls durch weitere funktionelle Gruppen substituiert sein, beispielsweise durch CN, SO₂R', SO₂OR' oder COOR'. R' bedeutet nicht, teilweise oder perfluoriertes C₁- bis C₆ Alkyl, C₃- bis C₇-Cycloalkyl, unsubstituiertes oder substituiertes Phenyl.

Bevorzugt sind die Alkylgruppen als Substituenten R und R¹ bis R⁶ sowie R^{1'} und R^{4'} der heterocyclischen Kationen der Formel (4) unterschiedlich von der Alkylgruppe des Anions im Onium Alkylsulfat.
Das erfindungsgemäß hergestellte Onium Alkylsulfat kann jedoch auch Alkylgruppen im Kation haben, die zu der Alkylgruppe im Anion gleich sind, die jedoch erfindungsgemäß nicht durch Alkylierung eingeführt wurden. Der Focus liegt dann auf der einfachen Reaktionsführung und des besonders niedrigen Halogenid-Gehalts im Endprodukt.

Der Substituent R in Formel (1) ist insbesondere jeweils unabhängig voneinander bevorzugt Methyl, Ethyl, Isopropyl, Propyl, Butyl, sek.-Butyl, tert.-Butyl, Pentyl, Hexyl, Octyl, Decyl oder Tetradecyl.

Bis zu vier Substituenten des Guanidinium-Kations [C(NR¹R²)(NR³R⁴)(NR⁵R⁶)]⁺ können auch paarweise derart verbunden sein, dass mono-, bi- oder polycyclische Kationen entstehen.
Ohne Einschränkung der Allgemeinheit sind Beispiele für solche Guanidinium-Kationen: 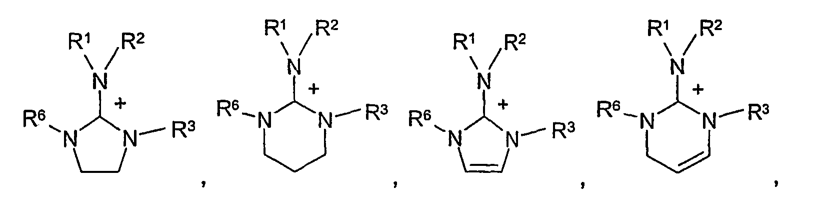 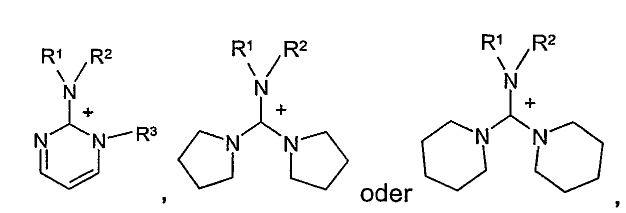 wobei die
Substituenten R¹ bis R³ und R⁶ eine zuvor angegebene oder besonders bevorzugte Bedeutung haben können.
Gegebenenfalls können die Carbocyclen oder Heterocyclen der zuvor angegebenen Guanidinium-Kationen noch durch C₁- bis C₆-Alkyl, C₁- bis C₆-Alkenyl, NO₂, F, Cl, Br oder I substituiert sein.

Bis zu vier Substituenten des Thiouroniumkations [(R¹R²N)-C(=SR⁷)(NR³R⁴)]⁺ können auch paarweise derart verbunden sein, dass mono-, bi- oder polycyclische Kationen entstehen.
Ohne Einschränkung der Allgemeinheit sind Beispiele für solche Kationen im folgenden angegeben: 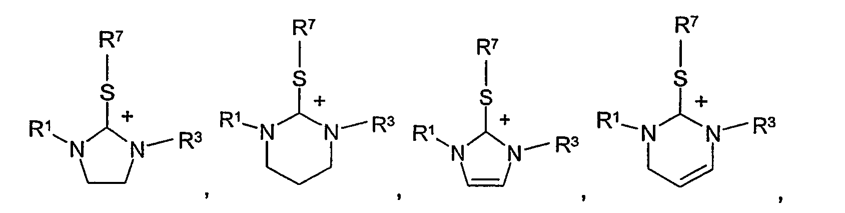 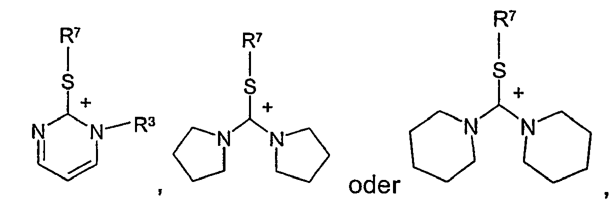 wobei die Substituenten
R¹, R³ und R⁷ eine zuvor angegebene oder besonders bevorzugte Bedeutung haben können.
Gegebenenfalls können die Carbocyclen oder Heterocyclen der zuvor angegebenen Thiouronium-Kationen noch durch C₁- bis C₆-Alkyl, C₁- bis C₆-Alkenyl, NO₂, F, Cl, Br, I, C₁-C₆-Alkoxy, SCF₃, SO₂CF₃ SO₂CH₃, COOR", SO₂NR"₂, SO₂X' oder SO₃R" substituiert sein, wobei X' F, Cl oder Br und R" ein nicht, teilweise oder perfluoriertes C₁- bis C₆-Alkyl oder C₃-bis C₇-Cycloalkyl wie für R' definiert bedeutet oder durch substituiertes oder unsubstituiertes Phenyl substituiert sein.

Die Substituenten R¹ bis R⁷ sind jeweils unabhängig voneinander bevorzugt eine geradkettige oder verzweigte Alkylgruppe mit 1 bis 10 C-Atomen. Die Substituenten R¹ und R², R³ und R⁴ und R⁵ und R⁶ in Verbindungen der Formeln (2) oder (3) können dabei gleich oder verschieden sein. Besonders bevorzugt sind R¹ bis R⁷ jeweils unabhängig voneinander Methyl, Ethyl, n-Propyl, Isopropyl, n-Butyl, tert.-Butyl oder sek.-Butyl, ganz besonders bevorzugt Methyl, Ethyl, n-Propyl, Isopropyl oder n-Butyl.

Als Substituenten R^{1'} bis R^{4'} von Verbindungen der Formel (4) kommen erfindungsgemäß dabei neben Wasserstoff bevorzugt in Frage: C₁- bis C₂₀-, insbesondere C₁- bis C₁₂-Alkylgruppen oder Aryl-C₁-C₆-alkyl.

Die Substituenten R^{1'} und R^{4'} sind jeweils unabhängig voneinander insbesondere bevorzugt Methyl, Ethyl, Isopropyl, Propyl, Butyl, sek.-Butyl, tert.-Butyl, Pentyl, Hexyl, Octyl, Decyl, Undecyl, Dodecyl oder Benzyl. Sie sind ganz besonders bevorzugt Methyl, Ethyl, n-Butyl, Hexyl oder Benzyl.
In Pyrrolidinium-, oder Piperidinium-Verbindungen sind die beiden Substituenten R^{1'} und R^{4'} bevorzugt unterschiedlich.

Der Substituent R^{2'} oder R^{3'} ist jeweils unabhänigig voneinander insbesondere Wasserstoff, Methyl, Ethyl, Isopropyl, Propyl, Butyl, sek.-Butyl oder tert.-Butyl. Besonders bevorzugt ist R^{2'} Wasserstoff, Methyl, oder Ethyl. Ganz besonders bevorzugt sind R^{2'} und R^{3'} Wasserstoff.

HetN⁺ der Formel (4) ist bevorzugt 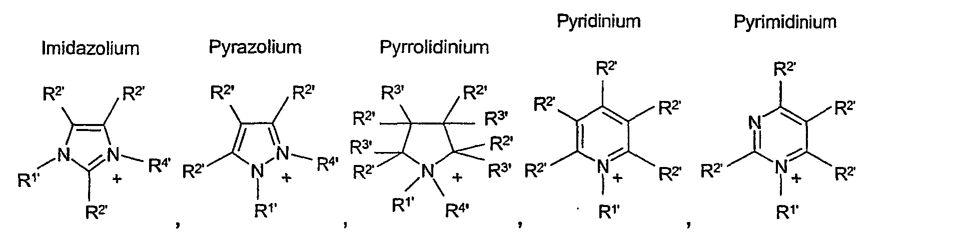 oder 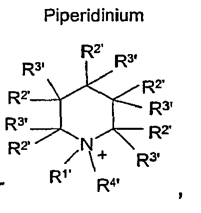 wobei die Substituenten R^{1'} bis R^{4'} jeweils unabhängig voneinander eine zuvor beschriebene Bedeutung haben.

HetN⁺ ist besonders bevorzugt Imidazolium, Pyrrolidinium oder Pyridinium, wie zuvor definiert, wobei die Substituenten R^{1'} bis R^{4'} jeweils unabhängig voneinander eine zuvor beschriebene Bedeutung haben.

Als symmetrisch substituiertes Dialkylsulfat wird bevorzugt ein Dialkylsulfat mit einer geradkettigen oder verzweigten Alkylgruppe mit 1-14 C-Atomen, bevorzugt mit 1-8 C-Atomen eingesetzt. Beispiele für symmetrisch substituierte Dialkylsulfate sind Dimethylsulfat, Diethylsulfat, Di-(n-propyl)sulfat, Di-(iso-propyl)sulfat, Di-(n-butyl)sulfat, Di-(sek-butyl)sulfat oder Di-(n-pentyl)sulfat, Di-(n-hexyl)sulfat, Di-(n-heptyl)sulfat oder Di-(n-octyl)sulfat.

Die eingesetzten symmetrischen Dialkylsulfate sind in der Regel kommerziell erhältlich oder können nach Syntheseverfahren hergestellt werden, wie sie aus der Literatur, z.B. in den Standardwerken wie Houben-Weyl, Methoden der organischen Chemie, Georg-Thieme-Verlag, Stuttgart oder Richard C. Larock, Comprehensive Organic Transformations, 2nd Edition, Wiley-VCH, New York, 1999 bekannt sind. Dabei kann man auch von an sich bekannten, hier nicht näher erwähnten Varianten Gebrauch machen.

Als unsymmetrisch substituiertes Dialkylsulfat wird bevorzugt ein Dialkylsulfat mit einer geradkettigen oder verzweigten Alkylgruppe mit 4 bis 20 C-Atomen und einer Methyl- oder Ethylgruppe als zweite Alkylgruppe, bevorzugt mit einer Alkylgruppe mit 4-8 C-Atomen, eingesetzt. Beispiele für unsymmetrisch substituierte Dialkylsulfate sind Methyl-butylsulfat, Ethylbutylsulfat, Methyl-pentylsulfat, Ethyl-pentylsulfat, Methyl-hexylsulfat, Ethylhexylsulfat, Methyl-heptylsulfat, Ethyl-heptylsulfat, Methyl-octylsulfat oder Ethyl-octylsulfat.

Die eingesetzten unsymmetrischen Dialkylsulfate können nach Syntheseverfahren hergestellt werden, wie sie aus der Literatur, z.B. in den Standardwerken wie Houben-Weyl, Methoden der organischen Chemie, Georg-Thieme-Verlag, Stuttgart oder Richard C. Larock, Comprehensive Organic Transformations, 2nd Edition, Wiley-VCH, New York, 1999 bekannt sind. Dabei kann man auch von an sich bekannten, hier nicht näher erwähnten Varianten Gebrauch machen.

Alkyl-trialkylsilyl-sulfate entsprechen der Formel Alkyl-O-SO₂-OSi(Alkyl')₃,
wobei die Alkylgruppe 1 bis 20 C-Atome und die Alkyl'-Gruppe 1 bis 4 C-Atome haben kann. Bevorzugt sind die Alkyl'-Gruppen gleich. Bevorzugt ist Alkyl' Methyl.

Die eingesetzten Alkyl-trialkylsilyl-sulfate können nach Syntheseverfahren hergestellt werden, wie sie aus der Literatur, z.B. in den Standardwerken wie Houben-Weyl, Methoden der organischen Chemie, Georg-Thieme-Verlag, Stuttgart oder Richard C. Larock, Comprehensive Organic Transformations, 2nd Edition, Wiley-VCH, New York, 1999) bekannt sind.

Dabei kann man auch von an sich bekannten, hier nicht näher erwähnten Varianten Gebrauch machen.

Alkyl-acyl-sulfate entsprechen der Formel Alkyl-O-SO₂-O-C(O)R^{F}, wobei die Alkylgruppe 1 bis 20 C-Atome haben kann und die R^{F}-Gruppe eine Perfluoralkylgruppe mit 1 bis 4 C-Atome bedeutet. Bevorzugt ist R^{F} Trifluormethyl.

Die eingesetzten Alkyl-acyl-sulfate können nach Syntheseverfahren hergestellt werden, wie sie aus der Literatur, z.B. in den Standardwerken wie Houben-Weyl, Methoden der organischen Chemie, Georg-Thieme-Verlag, Stuttgart oder Richard C. Larock, Comprehensive Organic Transformations, 2nd Edition, Wiley-VCH, New York, 1999 bekannt sind. Dabei kann man auch von an sich bekannten, hier nicht näher erwähnten Varianten Gebrauch machen.

Alkyl-sulfonyl-sulfate entsprechen der Formel Alkyl-O-SO₂-O-SO₂R^{F}, wobei die Alkylgruppe 1 bis 20 C-Atome haben kann und die R^{F'}-Gruppe eine Perfluoralkylgruppe mit 1 bis 4 C-Atomen, Cl oder F bedeutet. Bevorzugt ist R^{F'} F oder Trifluormethyl.

Die eingesetzten Alkyl-sulfonyl-sulfate können nach Syntheseverfahren hergestellt werden, wie sie aus der Literatur, z.B. in den Standardwerken wie Houben-Weyl, Methoden der organischen Chemie, Georg-Thieme-Verlag, Stuttgart oder Richard C. Larock, Comprehensive Organic Transformations, 2nd Edition, Wiley-VCH, New York, 1999 bekannt sind. Dabei kann man auch von an sich bekannten, hier nicht näher erwähnten Varianten Gebrauch machen.

Ein allgemeines Schema fasst das erfindungsgemäße Verfahren zusammen: 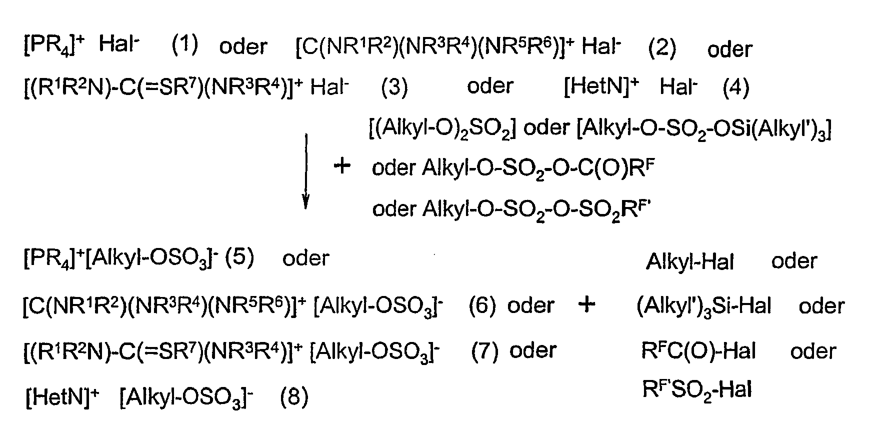 Die Substituenten R, R¹ bis R⁷ und HetN⁺ der Verbindungen der Formeln (1) bis (8) entsprechen den Bedeutungen, wie zuvor beschrieben.

Die Reaktion mit Dialkylsulfaten wird erfindungsgemäß bei Raumtemperatur, d.h. in der Regel bei Temperaturen zwischen 10° und 30°C durchgeführt. Die Umsetzung mit Alkyl-trialkylsilyl-sulfaten, Alkyl-acylsulfaten oder Alkyl-sulfonyl-sulfaten kann bei Temperaturen zwischen 0° und 200°C, bevorzugt bei 10° bis 100°, besonders bevorzugt bei 10° bis 50°C, ganz besonders bevorzugt bei Raumtemperatur durchgeführt, wobei die Temperaturen ab 50°C der Temperatur der Heizquelle, beispielsweise des Ölbads, entspricht. Es wird kein Lösungsmittel benötigt. Es können jedoch auch Lösungsmittel eingesetzt werden, beispielsweise Dimethoxyethan, Acetonitril, Aceton, Tetrahydrofuran, Dimethylformamid, Dimethylsulfoxid, Dioxan, Propionitril oder Gemische untereinander.

Die Umsetzung wird mit einem Überschuss oder equimolarer Menge an Dialkylsulfat, Alkyl-trialkylsilyl-sulfat, Alkyl-acyl-sulfat oder Alkyl-sulfonyl-sulfat durchgeführt.

Die beschriebene Methode ist auch für die Reinigung der Onium-Salze geeignet. Das bedeutet, dass der durch Halogenid-lonen verunreinigten ionischen Flüssigkeit, beispielsweise 1-Butyl-3-methylimidazolium Methylsulfat, verunreinigt mit 1-Butyl-3-methylimidazoliumchlorid, ein entsprechender erfindungsgemäßer Ester oder ein Silylester der Säure des Anions, beispielsweise mit Dimethylsulfat zugegeben wird. Die Verunreinigung reagiert ab und man kommt zur halogenidreduzierten ionischen Flüssigkeit.

Gegenstand der Erfindung ist ebenfalls ein Eintopfverfahren zur Herstellung von Onium Alkylsulfaten, insbesondere von Alkylsulfaten, wobei die Alkylgruppe 4 bis 20 C-Atome hat, besonders bevorzugt 4 bis 14 C-Atome hat, **dadurch gekennzeichnet, dass** ein Onium Halogenid mit einem symmetrisch substituierten Dialkylsulfat, wobei die Alkylgruppe 1 bis 3 C-Atome haben kann und mit einem Alkohol mit 4 bis 20 C-Atomen umgesetzt wird.

Die Nebenprodukte Alkylhalogenid mit 1 bis 3 C-Atomen sowie der Alkohol mit 1 bis 3 C-Atomen können leicht entfernt werden.

Die Umsetzung wird bei Temperaturen zwischen zwischen 0° und 200°C, bevorzugt bei 10° bis 100°, besonders bevorzugt bei 10° bis 60°C und Anwendung von Vakuum durchgeführt, wobei die Temperaturen ab 60°C der Temperatur der Heizquelle, beispielsweise des Ölbads, entspricht.

Besonders bevorzugt wird die Eintopfreaktion mit dem symmetrischen Dimethylsulfat und den Alkoholen Hexanol, Heptanol oder Octanol, ganz besonders bevorzugt Octanol durchgeführt.

Gegenstand der Erfindung sind auch die Verbindungen Trialkylsilyloctylsulfat, wobei die Alkylgruppe der Trialkylsilylgruppe 1 bis 4 C-Atome haben kann. Bevorzugte Trialkylsilyloctylsulfate sind Verbindungen, deren Alkylgruppe in der Trialkylsilylgruppe gleich ist. Besonders bevorzugt ist Trimethylsilyl-octyl-sulfat oder Triethylsilyl-octyl-sulfat, ganz besonders bevorzugt Trimethylsilyl-octyl-sulfat.

Diese Verbindungen eigenen sich hervorragend für den Einsatz im erfindungsgemäßen Verfahren, d.h. zur Einführung von Octylsulfat-Anionen in ionische Flüssigkeiten.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

Es versteht sich für den Fachmann von selbst, dass in den vorab und nachfolgend genannten Verbindungen Substituenten wie beispielsweise H, N, 0, Cl, F durch die entsprechenden Isotope ersetzt sein können.

Die NMR-Spektren wurden an Lösungen in deuterierten Lösungsmitteln bei 20°C an einem Bruker ARX 400 Spektrometer mit einem 5 mm Breitbandkopf ¹H/BB mit Deuterium Lock gemessen, falls nicht in den Beispielen angegeben. Die Messfrequenzen der verschiedenen Kerne sind: ¹H: 400,13 MHz und ¹⁹F: 376,50 MHz. Die Methode der Referenzierung wird bei jedem Spektrum bzw. bei jedem Datensatz separat angegeben.

### Beispiel 1:

### Synthese von 1-Butyl-3-methylimidazolium Ethylsulfat

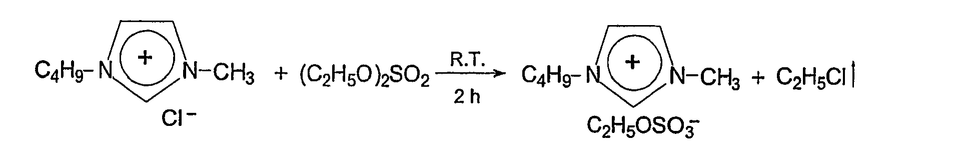

Eine Mischung von 3.62 g (20.7 mmol) 1-Butyl-3-methylimidazoliumchlorid und 3.19 g (20.7 mmol) Diethylsulfat werden bei Raumtemperatur für zwei Stunden gerührt. NMR-Messungen zeigen die Vollständigkeit der Reaktion.

Der Rückstand wird für 30 Minuten im Vakuum bei 13.3 Pa und 60°C (Ölbadtemperatur) getrocknet. Man erhält 5.47 g 1-butyl-3-methylimidazolium Ethylsulfat in annähernd quantitativer Ausbeute.
¹H NMR (Referenz: TMS ; CD₃CN), ppm: 0.92 t (CH₃); 1.18 t (CH₃); 1.31 m (CH₂); 1.81 m (CH₂); 3.86 s (CH₃); 3.88 q (CH₂); 4.16 t (CH₂); 7.42 d,d (CH); 7.45 d,d (CH); 8.88 br. s. (CH); ³J_{H,H} = 7.4 Hz ; ³J_{H,H} = 7.2; ³J_{H,H} = 7.1 Hz; J_{H,H} = 1.7 Hz.

### Beispiel 2:

### Synthese von 1-Hexyl-3-methyfimidazolium Methylsulfat

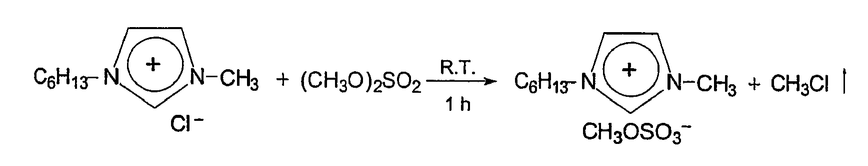

Analog zu Beispiel 1 werden 1.51 g (7.45 mmol) 1-Hexyl,-3-methylimidazoliumchlorid und 1.02 g (8.09 mmol) Dimethylsulfat für eine Stunde gerührt und im Vakuum bei 13.3 Pa und 120°C (Ölbadtemperatur) getrocknet. Man erhält 2.06 g 1-Hexyl-3-methylimidazolium Methylsulfat in annähernd quantitativer Ausbeute.
¹H NMR (Referenz: TMS ; CD₃CN), ppm: 0.86 m (CH₃); 1.28 m (3CH₂); 1.81 m (CH₂); 3.50 s (OCH₃); 3.84 s (CH₃); 4.13 t (CH₂); 7.39 d,d (CH); 7.42 d,d (CH); 8.81 br. s. (CH); ³J_{H,H} = 7.1 Hz; J_{H,H} = 1.5 Hz.

### Beispiel 3:

### Synthese von 1-Butyl-3-methylimidazolium Methylsulfat

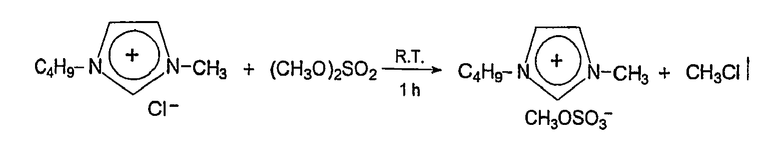

Analog zu Beispiel 1 werden 1.36 g (7.79 mmol) 1-Butyl-3-methylimidazoliumchlorid und 0.99 g (7.95 mmol) Dimethylsulfat für eine Stunde gerührt und im Vakuum bei 13.3 Pa und 120°C (Ölbadtemperatur) getrocknet. Man erhält 1.95 g 1-Butyl-3-methylimidazolium Methylsulfat in annähernd quantitativer Ausbeute.
¹H NMR (Referenz: TMS ; CD₃CN), ppm: 0.90 t (CH₃); 1.29 m (CH₂); 1.79 m (CH₂); 3.50 s (OCH₃); 3.84 s (CH₃); 4.15 t (CH₂); 7.40 d,d (CH); 7.43 d,d (CH); 8.91 br. s. (CH); ³J_{H,H} = 7.4 Hz ; ³J_{H,H} = 7.1 Hz; J_{H,H} = 1.7 Hz.

### Beispiel 4:

### Analog zu Beispiel 3 werden

1,3-Dimethylimidazoliumchlorid mit Dimethylsulfat zu
   1,3-Dimethylimidazolium Methylsulfat;
1,3-Dibutylimidazoliumchlorid mit Dimethylsulfat zu
   1,3-Dibutylimidazolium Methylsulfat;
1-Ethyl-3-methylimidazoliumchlorid mit Dimethylsulfat zu
   1-Ethy1-3-methylimidazolium Methylsulfat;
1-Ethyl-3-methylimidazoliumchlorid mit Diethylsulfat zu
   1-Ethyl-3-methylimidazolium Ethylsulfat;
1-Ethyl-3-methylimidazoliumchlorid mit Dibutylsulfat zu
   1-Ethyl-3-methylimidazolium Butylsulfat;
1-Ethyl-3-methylimidazoliumchlorid mit Dihexylsulfat zu
   1-Ethyl-3-methylimidazolium Hexylsulfat;
1-Ethyl-3-methylimidazoliumchlorid mit Dioctylsulfat zu
   1-Ethyl-3-methylimidazolium Octylsulfat;
1-Butyl-3-methylimidazoliumchlorid mit Dioctylsulfat zu
   1-Butyl-3-methylimidazolium Octylsulfat;
3-Methyl-1-octylimidazoliumchlorid mit Dioctylsulfat zu
   3-Methyl-1-octylimidazolium Octylsulfat;
3-Methyl-1-octylimidazoliumchlorid mit Dimethylsulfat zu
   3-Methyl-1-octylimidazolium Methylsulfat;
1-Benzyl-3-methylimidazoliumchlorid mit Dimethylsulfat zu
   1-Benzyl-3-methylimidazolium Methylsulfat;
1-Ethyl-2,3-dimethylimidazoliumchlorid mit Dimethylsulfat zu
   1-Ethyl-2,3-dimethylimidazolium Methylsulfat;
1-Butyl-2,3-dimethylimidazoliumchlorid mit Dimethylsulfat zu
   1-Butyl-2,3-dimethylimidazolium Methylsulfat;
1-Butyl-2,3-dimethylimidazoliumchlorid mit Dioctylsulfat zu
   1-Butyl-2,3-dimethylimidazolium Octylsulfat umgesetzt.

### Beispiel 5:

### Synthese von Trihexyl-tetradecyl-phosphonium Methylsulfat

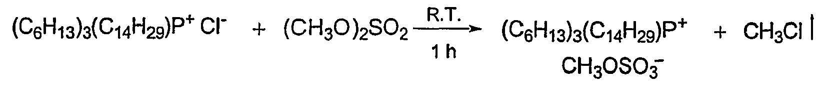

Eine Mischung von 1.72 g (3.31 mmol) Trihexyl-tetradecyl-phosphoniumchlorid und 0.51 g (4.04 mmol) Dimethylsulfat wird eine Stunde bei Raumtemperatur gerührt. NMR-Messungen zeigen die Vollständigkeit der Reaktion an. Der Rückstand wird bei einem Vakuum von 13.3 Pa und 120°C (Ölbadtemperatur) für 30 Minuten getrocknet. Man erhält 1.96 g Trihexyl-tetradecyl-phosphonium Methylsulfat in annähernd quantitativer Ausbeute.
¹H NMR (Referenz: TMS ; CD₃CN), ppm: 0.85-0.93 m (4CH₃), 1.24-1.37 m (16CH₂), 1.37-1.59 m (8CH₂), 2.02-2.12 m (4CH₂); 3.54 s (OCH₃). ³¹P {¹H} NMR (Referenz: 85% H₃PO₄ - extern; CD₃CN), ppm: 33.3.

### Beispiel 6:

Analog zu Beispiel 5 wird Tributyl-methyl-phosphoniumchlorid mit Diethylsulfat zu Tributyl-methyl-phosphonium Ethylsulfat umgesetzt.

### Beispiel 7:

### Synthese von 1-Ethylpyridinium Methylsulfat

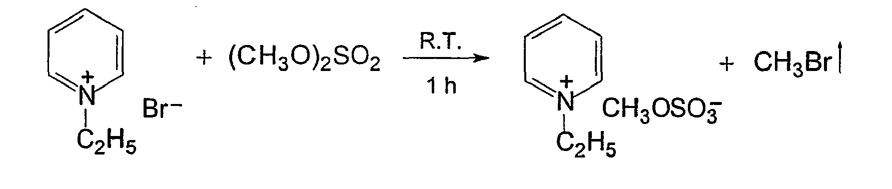

Eine Mischung von 1.96 g (10.4 mmol) 1-Ethyl-pyridiniumbromid und 1.31 g (10.4 mmol) Dimethylsulfat wird eine Stunde bei Raumtemperatur gerührt. NMR-Messungen zeigen die Vollständigkeit der Reaktion an. Der Rückstand wird bei einem Vakuum von 13.3 Pa und 120°C (Ölbadtemperatur) für 30 Minuten getrocknet. Man erhält 2.28 g 1-Ethylpyridinium Methylsulfat in annähernd quantitativer Ausbeute.
¹H NMR (Referenz: TMS ; CD₃CN), ppm: 1.57 t (CH₃); 3.50 s (OCH₃); 4.63 q (CH₂); 8.04 m (2CH) ; 8.50 t (CH); 8.92 d (2CH); ³J_{H,H} = 7.1 Hz ; ³J_{H,H} = 7.9 Hz ; ³J_{H,H} = 6.1 Hz.

### Beispiel 8:

### Synthese von 1-Butylpyridinium Methylsulfat

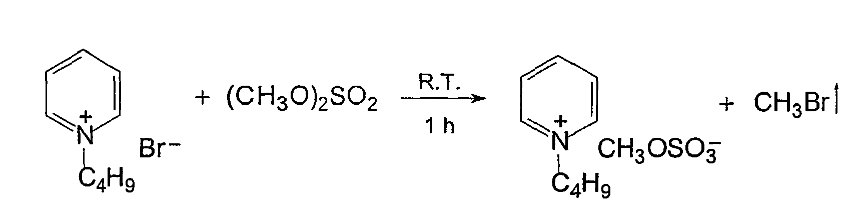

Eine Mischung von 1.22 g (5.65 mmol) 1-Butyl-pyridiniumbromid und 0.95 g (7.53 mmol) Dimethylsulfat wird eine Stunde bei Raumtemperatur gerührt. NMR-Messungen zeigen die Vollständigkeit der Reaktion an. Der Rückstand wird bei einem Vakuum von 13.3 Pa und 120°C (Ölbadtemperatur) für 30 Minuten getrocknet. Man erhält 1.39 g 1-Butylpyridinium Methylsulfat in annähernd quantitativer Ausbeute.
¹H NMR (Referenz: TMS; CD₃CN), ppm: 0.92 t (CH₃); 1.34 m (CH₂); 1.93 m (CH₂); 3.50 s (OCH₃); 4.58 t (CH₂); 8.04 m (2CH) ; 8.50 t (CH); 8.88 d (2CH); ³J_{H,H} = 7.3 Hz ; ³J_{H,H} = 7.6 Hz ; ³J_{H,H} = 7.9 Hz ; ³J_{H,H} = 6.6 Hz.

### Analog hierzu werden

1-Butyl-3-methylpyridiniumbromid mit Dimethylsulfat zu
   1-Butyl-3-methylpyridinium Methylsulfat;
1-Butyl-3-ethylpyridiniumbromid mit Dimethylsulfat zu
   1-Butyl-3-ethylpyridinium bromid Methylsulfat;
1-Butyl-4-methylpyridiniumchlorid mit Dimethylsulfat zu
   1-Butyl-4-methylpyridinium Methylsulfat oder
1-Butyl-4-ethylpyridiniumchlorid mit Dimethylsulfat zu
   1-Butyl-4-ethylpyridinium Methylsulfat umgesetzt.

### Beispiel 9:

### Synthese von 1-Ethyl-1-methylpyrrolidinium Ethylsulfat

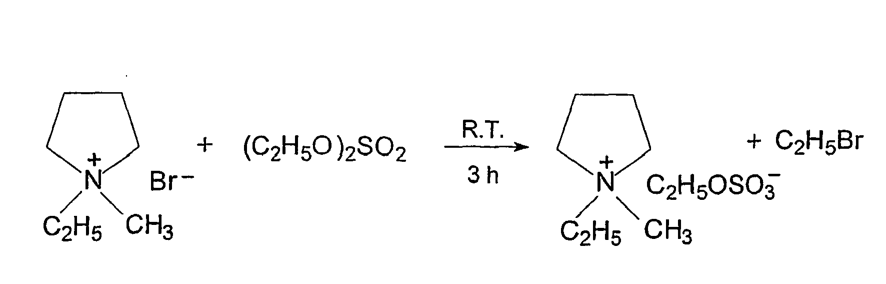

Eine Mischung von 2.35 g (12.11 mmol) 1-Ethyl-1-methyl-pyrrolidinium-bromid und 1.87 g (12.13 mmol) Diethylsulfat wird drei Stunden bei Raumtemperatur gerührt. NMR-Messungen zeigen die Vollständigkeit der Reaktion an. Der Rückstand wird bei einem Vakuum von 13.3 Pa bei Raumtemperatur für eine Stunde getrocknet. Man erhält 2,89 g 1-Ethyl-1-methylpyrrolidinium Ethylsulfat in annähernd quantitativer Ausbeute.
Smp.: 35-36°C
¹H NMR (Referenz: TMS; CD₃CN), ppm: 1.16 t (CH₃); 1.31 t,m (CH₃); 2.14 m (2CH₂); 2.95 s (CH₃); 3.37 q (CH₂); 3.44 m (2CH₂); 3.84 q (CH₂); ³J_{H,H} = 7.1 Hz.

### Analog hierzu werden

1-Butyl-1-methylpyrrolidiniumbromid mit Diethylsulfat zu
   1-Butyl-1-methylpyrrolidinium Ethylsulfat umgesetzt.

### Beispiel 10:

### Synthese von N,N,N',N'-Tetramethyl-N"-ethyl-guanidinium Methylsulfat

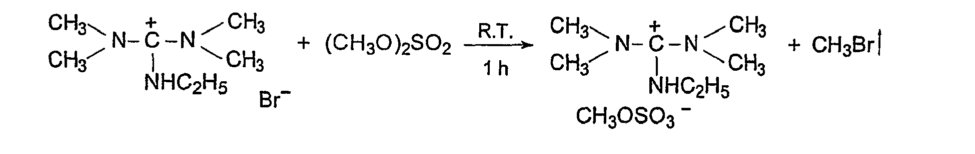

Eine Mischung von 2.59 g (11.56 mmol) N,N,N',N'-Tetramethyl-N"-ethyl-guanidiniumbromid und 1.46 g (11.58 mmol) Dimethylsulfat wird eine Stunde bei Raumtemperatur gerührt. NMR-Messungen zeigen die Vollständigkeit der Reaktion an. Der Rückstand wird bei einem Vakuum von 13.3 Pa bei Raumtemperatur für zwei Stunden getrocknet. Man erhält 2.95 g N,N,N',N'-Tetramethyl-N"-ethyl-guanidinium Methylsulfat als viskose Flüssigkeit in annähernd quantitativer Ausbeute.
¹H NMR (Referenz: TMS; CD₃CN), ppm: 1.11 t (CH₃); 2.86 br.s ; 2.88 br.s ; 2.92 s (4CH₃); 3.20 m (CH₂); 3.49 s (OCH₃); 6.90 br.s (NH); ³J_{H,H} = 7.1 Hz.

### Analog hierzu werden

Guanidiniumchlorid mit Dimethylsulfat zu
   Guanidinium Methylsulfat;
Guanidiniumchlorid mit Diethylsulfat zu
   Guanidinium Ethylsulfat oder
N,N,N',N'-Tetramethyl-N",N"-diethylguanidiniumbromid mit Dimethylsulfat zu
   N,N,N',N'-Tetramethyl-N",N"-diethylguanidinium Methylsulfat umgesetzt.

### Beispiel 11:

### Synthese von 1-Ethyl-3-methylimidazolium Methylsulfat

### a) Synthese von 1-Ethyl-3-methylimidazoliumbromid

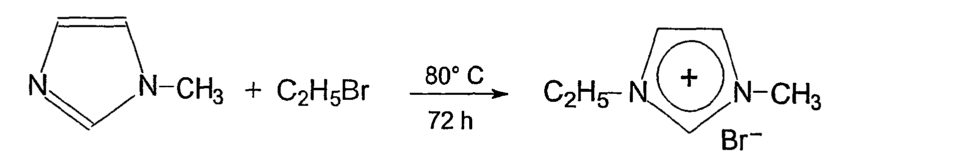

Es werden 111.43 g (1.36 mol) Methylimidazol und 160 g (1.47 mol) Bromethan vermischt und anschließend 400 ml Isopropanol zugegeben. Die Reaktionsmischung wird unter Rühren für 72 Stunden erhitzt, wobei die Ölbadtemperatur 80°C beträgt. Isopropanol wird danach abdestilliert und der Rückstand im Vakuum bei 13.3 Pa und 100°C Ölbadtemperatur für zwei Stunden getrocknet. Man erhält 258.6 g 1-Ethyl-3-methylimidazoliumbromid, das entspricht einer Ausbeute von 99.7 %.
Smp.: 73-74°C.
¹H NMR (Referenz: TMS; CD₃CN), ppm: 1.44 t (CH₃); 3.88 s (CH₃); 4.23 q (CH₂); 7.49 m (CH); 7.56 m (CH); 9.45 br. s. (CH); ³J_{H,H} = 7.2 Hz.

### b) Synthese von 1-Ethyl-3-methylimidazolium Methylsulfat

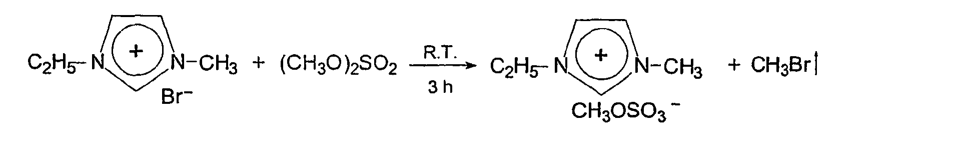

Zu 203.26 g (1.064 mol) 1-Ethyl-3-methyl-imidazoliumbromid werden 134.17 g (1.064 mol) Dimethylsulfat zugegeben. Die Reaktionsmischung wrd drei Stunden bei Raumtemperatur gerührt, bis sich das Bromid vollständig gelöst hat. Anschließend wird das flüssige Produkt im Vakuum bei 13.3 Pa und Raumtemperatur für drei Stunden getrocknet. Man erhält 236.4 g 1-Ethyl-3-methyl-imidazolium Methylsulfat, das entspricht einer annähernd quantitativen Ausbeute.
¹H NMR (Referenz: TMS; CD₃CN), ppm: 1.44 t (CH₃); 3.50 s (OCH₃); 3.84 s (CH₃); 4.18 q (CH₂); 7.40 m (CH); 7.45 m (CH); 8.84 br. s. (CH); ³J_{H,H} = 7.3 Hz.

### Beispiel 12:

### Synthese von N,N,N',N'-Tetramethyl-S-methylthiouronium Ethylsulfat

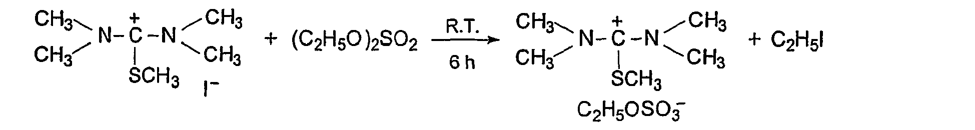

Eine Mischung von 2.25 g (8.21 mmol) N,N,N',N'-Tetramethyl-S-methylthiouroniumiodid und 1.27 g (8.24 mmol) Diethylsulfat wird für 6 Stunden bei Raumtemperatur gerührt. Eine NMR-Messung zeigt die Vollständigkeit der Reaktion an. Der Rückstand wird bei Raumtemperatur im Vakuum von 13.3 Pa 1 Stunde getrocknet. Man erhält 2.16 g N,N,N',N'-Tetramethyl-S-methylthiouronium Ethylsulfat, das entspricht einer Ausbeute von 96.6 %.
¹H NMR (Referenz: TMS; CD₃CN), ppm: 1.50 t (CH₃); 2.49 s (SCH₃); 3.21 s (4CH₃); 3.82 q (CH₂); ³J_{H,H} = 7.1 Hz.

### Analog hierzu werden

N,N,N',N'-Tetramethyl-S-ethylthiouroniumiodid mit Diethylsulfat zu
   N,N,N',N'-Tetramethyl-S-ethylthiouronium Ethylsulfat;
N,N,N',N'-Tetramethyl-S-propylthiouroniumiodid mit Dimethylsulfat zu
   N,N,N',N'-Tetramethyl-S-propylthiouronium Methylsulfat;
N,N,N',N'-Tetramethyl-S-butylthiouroniumiodid mit Diethylsulfat zu
   N,N,N',N'-Tetramethyl-S-butylthiouronium Ethylsulfat;
N,N,N',N'-Tetramethyl-S-octylthiouroniumiodid mit Diethylsulfat zu
   N,N,N',N'-Tetramethyl-S-octylthiouronium Ethylsulfat;
N,N,N',N'-Tetraethyl-S-methylthiouroniumiodid mit Diethylsulfat zu
   N,N,N',N'-Tetraethyl-S-methylthiouronium Ethylsulfat;
N,N,N',N'-Tetraethyl-S-ethylthiouroniumiodid mit Diethylsulfat zu
   N,N,N',N'-Tetraethyl-S-ethylthiouronium Ethylsulfat;
N,N,N',N'-Tetraethyl-S-propylthiouroniumiodid mit Dimethylsulfat zu
   N,N,N',N'-Tetraethyl-S-propylthiouronium Methylsulfat;
N,N,N',N'-Tetraethyl-S-butylthiouroniumiodid mit Diethylsulfat zu
   N,N,N',N'-Tetraethyl-S-butylthiouronium Ethylsulfat;
N,N,N',N'-Tetraethyl-S-octylthiouroniumiodid mit Diethylsulfat zu
   N,N,N',N'-Tetraethyl-S-octylthiouronium Ethylsulfat;
N,N-Dimethyl-N',N'-diethyl-S-methylthiouroniumiodid mit Diethylsulfat zu
   N,N-Dimethyl-N',N'-diethyl-S-methylthiouronium Ethylsulfat;
N,N-Dimethyl-N',N'-diethyl-S-ethylthiouroniumiodid mit Diethylsulfat zu
   N,N-Dimethyl-N',N'-diethyl-S-ethylthiouronium Ethylsulfat;
N,N-Dimethyl-N',N'-diethyf-S-propylthiouroniumiodid mit Dimethylsulfat zu
   N,N-Dimethyl-N',N'-diethyl-S-propylthiouronium Methylsulfat;
N,N-Dimethyl-N',N'-diethyl-S-butylthiouroniumiodid mit Diethylsulfat zu
   N,N-Dimethyl-N',N'-diethyl-S-butylthiouronium Ethylsulfat oder
N,N-Dimethyl-N',N'-diethyl-S-octylthiouroniumiodid mit Diethylsulfat zu
   N,N-Dimethyl-N',N'-diethyl-S-octylthiouronium Ethylsulfat umgesetzt.

### Beispiel 13:

### Synthese von 1-Butyl-3-methylimidazolium methylsulfat

### a) Synthese von Trimethylsilylmethylsulfat

(CH₃)₃SiOSO₂Cl + CH₃OH → CH₃OSO₂OSi(CH₃)₃ + HCl↑

Zu 3.3 g (17.49 mmol) Trimethylsilylester der Chlorsulfonsäure werden 0.56 g (17.48 mmol) Methanol innerhalb von 10 Minuten unter Rühren und Temperaturkontrolle zugegeben. Alle flüchtigen Produkte werden im Vakuum von 13 Pa bei Raumtemperatur entfernt. 1.21 g Trimethylchlorsilan werden zugegeben und die Reaktionsmischung wird 30 Minuten bei 70°C Ölbadtemperatur erhitzt. Die Mischung wird im Vakuum von 13 Pa fraktioniert destilliert. Man erhält 2.24 g Trimethylsilylmethylsulfat vom Siedepunkt 63-64°C. Die Ausbeute entspricht 69.9 %.
¹H NMR (Referenz: TMS; CD₃CN), ppm: 0.41 s [Si(CH₃)₃]; 3.92 s (CH₃).

### b) Synthese von 1-Butyl-3-methylimidazolium Methylsulfat

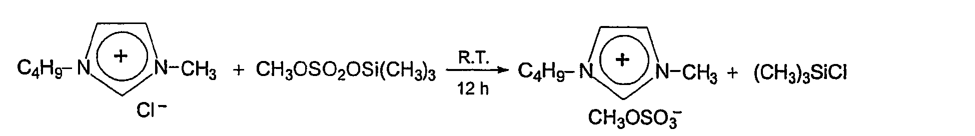

Eine Mischung von 0.91 g (5.21 mmol) 1-Butyl-3-methylimidazoliumchlorid und 0.96 g (5.21 mmol) Trimethylsilylmethylsulfat wird für 12 Stunden bei Raumtemperatur gerührt. Eine NMR-Messung zeigt die Vollständigkeit der Reaktion. Der Rückstand wird im Vakuum bei 13.3 Pa und einer Ölbadtemperatur von 60°C eine Stunde getrocknet. Man erhält 1.30 g 1-Butyl-3-methylimidazolium Methylsulfat. Die Ausbeute ist annähernd quantitativ.
¹H NMR (Referenz: TMS; CD₃CN), ppm: 0.92 t (CH₃); 1.31 m (CH₂); 1.81 m (CH₂); 3.52 s (OCH₃); 3.86 s (CH₃); 4.16 t (CH₂); 7.43 d,d (CH); 7.47 d,d (CH); 8.90 br. s. (CH); ³J_{H,H} = 7.4 Hz; ³J_{H,H} = 7.3 Hz; J_{H,H} = 1.7 Hz.

### Beispiel 14:

### Synthese 1-Butyl-3-methylimidazolium Octylsulfat

### a) Synthese von Trimethylsilyloctylsulfat

(CH₃)₃SiOSO₂Cl + C₈H₁₇OH → C₈H₁₇OSO₂OSi(CH₃)₃ + HCl↑

Zu 1.70 g (9.01 mmol) Trimethylsilylester der Chlorsulfonsäure werden 1.17 g (8.98 mmol) Octanol zugegeben. Die Reaktionsmischung wird 30 Minuten bei Raumtemperatur gerührt und anschließend alle flüchtigen Produkte im Vakuum von 13 Pa bei Raumtemperatur entfernt. 1.12 g Trimethylchlorsilan werden zugegeben und die Reaktionsmischung wird 30 Minuten bei 70°C Ölbadtemperatur erhitzt. Die Mischung wird im Vakuum von 13 Pa fraktioniert destilliert. Man erhält 2.48 g Trimethylsilyloctylsulfat vom Siedepunkt 132°C. Die Ausbeute entspricht 57.2 %.
¹H NMR (Referenz: TMS; CD₃CN), ppm: 0.40 s [Si(CH₃)₃]; 0.90 m (CH₃); 1.30 m (5CH₂); 1.73 m (CH₂); 4.24 t (CH₂); ³J_{H,H} = 6.5 Hz.

### b) Synthese von 1-Butyl-3-methylimidazolium Octylsulfat

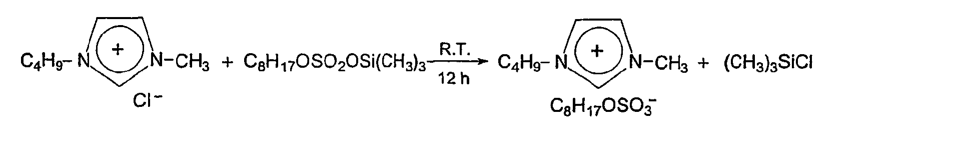

Eine Mischung von 0.358 g (2.05 mmol) 1-Butyl-3-methylimidazoliumchlorid und 0.58 g (2.06 mmol) Trimethylsilyloctylsulfat werden bei Raumtemperatur für 12 Stunden gerührt. NMR-Messungen zeigen die Vollständigkeit der Reaktion. Der Rückstand wird im Vakuum bei 13.3 Pa und 60°C Ölbadtemperatur für 1 Stunde getrocknet. Man erhält 0.71 g 1-Butyl-3-methylimidazolium Octylsulfat. Die Ausbeute ist annähernd quantitativ.
¹H NMR (Referenz: TMS ; CD₃CN), ppm: 0.87 t (CH₃); 0.92 t (CH₃); 1.27 m (5CH₂); 1.31 m (CH₂); 1.54 m (CH₂); 1.81 m (CH₂); 3.81 t (CH₂); 3.87 s (NCH₃); 4.19 t (CH₂); 7.46 d,d (CH); 7.49 d,d (CH); 9.16 br. s. (CH); ³J_{H,H} = 7.4 Hz; ³J_{H,H} = 7.3 Hz; ³J_{H,H} = 6.7 Hz; J_{H,H} = 1.7 Hz.

## Patentansprüche

1. Verfahren zur Herstellung von Onium Alkylsulfaten durch Umsetzung eines Onium Halogenids mit einem symmetrisch substituierten Dialkylsulfat, wobei die Alkylgruppe 1 bis 14 C-Atome haben kann, mit einem unsymmetrisch substituierten Dialkylsulfat, wobei eine Alkylgruppe 4 bis 20 C-Atome haben kann und die zweite Alkylgruppe Methyl oder Ethyl bedeutet, mit einem Alkyl-trialkylsilyl-sulfat, mit einem Alkyl-acyl-sulfat oder mit einem Alkyl-sulfonyl-sulfat, wobei die Umsetzung mit einem Dialkylsulfat bei Raumtemperatur stattfindet.

2. Verfahren nach Anspruch 1, **dadurch gekennzeichnet, dass** die Umsetzung mit einem symmetrisch substituierten Dialkylsulfat, wobei die Alkylgruppe 1 bis 14 C-Atome haben kann, bei Raumtemperatur stattfindet.

3. Verfahren nach Anspruch 1, **dadurch gekennzeichnet, dass** die Umsetzung mit einem unsymmetrisch substituierten Dialkylsulfat, wobei eine Alkylgruppe 4 bis 20 C-Atome haben kann und die zweite Alkylgruppe Methyl oder Ethyl bedeutet, bei Raumtemperatur stattfindet.

4. Verfahren nach Anspruch 1, **dadurch gekennzeichnet, dass** die Umsetzung mit einem Alkyl-trialkylsilyl-sulfat stattfindet.

5. Verfahren nach Anspruch 1, **dadurch gekennzeichnet, dass** die Umsetzung mit einem Alkyl-acyl-sulfat stattfindet.

6. Verfahren nach Anspruch 1, **dadurch gekennzeichnet, dass** die Umsetzung mit einem Alkyl-sulfonyl-sulfat stattfindet.

7. Verfahren nach einem oder mehreren der Ansprüche 1 bis 6, **dadurch gekennzeichnet, dass** das Halogenid ein Phosphoniumchlorid oder - bromid, ein Guanidiniumchlorid oder -bromid, ein Thiouroniumchlorid, - bromid oder -iodid oder Chlorid oder Bromid mit einem heterocyclischem Kation ist.

8. Verfahren nach einem oder mehreren der Ansprüche 1 bis 7, **dadurch gekennzeichnet, dass** das Halogenid der Formel (1) entspricht,
[PR₄]⁺ Hal⁻ (1),
wobei
Hal Cl oder Br und
R jeweils unabhängig voneinander
H, wobei nicht alle Substituenten R gleichzeitig H sein dürfen, geradkettiges oder verzweigtes Alkyl mit 1-20 C-Atomen, geradkettiges oder verzweigtes Alkenyl mit 2-20 C-Atomen und einer oder mehreren Doppelbindungen bedeutet,
wobei ein oder mehrere R teilweise oder vollständig mit Halogenen, insbesondere -F und/oder -Cl, oder teilweise mit -NO₂ substituiert sein können, wobei jedoch nicht alle vier oder drei R vollständig mit Halogenen substituiert sein dürfen.

9. Verfahren nach einem oder mehreren der Ansprüche 1 bis 7, **dadurch gekennzeichnet, dass** das Halogenid der Formel (2) entspricht,
[C(NR¹R²)(NR³R⁴)(NR⁵R⁶)]⁺ Hal⁻ (2),
wobei . Hal Cl oder Br und
R¹ bis R⁶ jeweils unabhängig voneinander
Wasserstoff,
geradkettiges oder verzweigtes Alkyl mit 1 bis 20 C-Atomen,
geradkettiges oder verzweigtes Alkenyl mit 2-20 C-Atomen und einer
oder mehreren Doppelbindungen, bedeutet,
wobei ein oder mehrere der Substituenten R¹ bis R⁶ teilweise oder vollständig mit Halogenen, insbesondere -F und/oder -Cl, oder teilweise mit -NO₂, substituiert sein können, wobei jedoch nicht alle Substituenten an einem N-Atom vollständig mit Halogenen substituiert sein dürfen und
wobei die Substituenten R¹ bis R⁶ paarweise durch Einfach- oder Doppelbindung miteinander verbunden sein können.

10. Verfahren nach einem oder mehreren der Ansprüche 1 bis 7, **dadurch gekennzeichnet, dass** das Halogenid der Formel (3) entspricht,
[(R¹R²N)-C(=SR⁷)(NR³R⁴)]⁺ Hal⁻ (3)
beschrieben werden,
wobei
Hal Cl, Br oder I und
R¹ bis R⁷ jeweils unabhängig voneinander
Wasserstoff, wobei Wasserstoff für R⁷ ausgeschlossen wird, geradkettiges oder verzweigtes Alkyl mit 1 bis 20 C-Atomen, geradkettiges oder verzweigtes Alkenyl mit 2-20 C-Atomen und einer oder mehreren Doppelbindungen,
bedeutet,
wobei ein oder mehrere der Substituenten R¹ bis R⁷ teilsweise oder vollständig mit Halogenen, insbesondere -F und/oder -Cl, oder teilweise mit -NO₂ substituiert sein können, wobei jedoch nicht alle Substituenten an einem N-Atom vollständig mit Halogenen substituiert sein dürfen und
wobei die Substituenten R¹ bis R⁷ paarweise durch Einfach- oder Doppelbindung miteinander verbunden sein können.

11. Verfahren nach einem oder mehreren der Ansprüche 1 bis 7,
**dadurch gekennzeichnet, dass** das Halogenid der Formel (4) entspricht,
[HetN]⁺ Hal⁻ (4),
wobei
Hal Cl oder Br und
HetN⁺ ein heterocyclisches Kation, ausgewählt aus der Gruppe 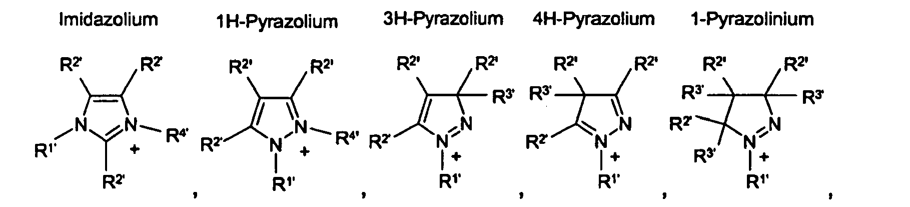 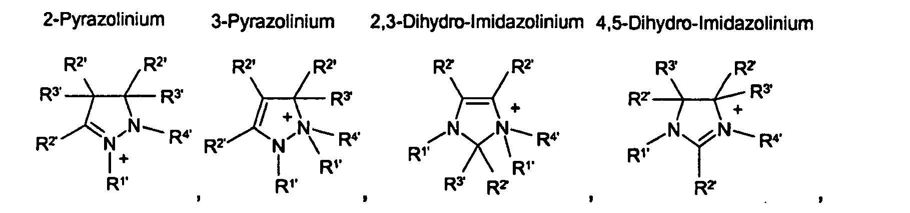 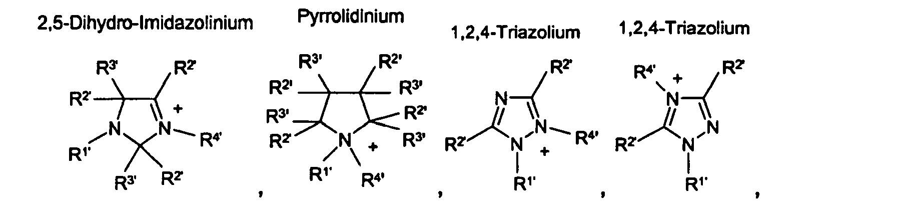 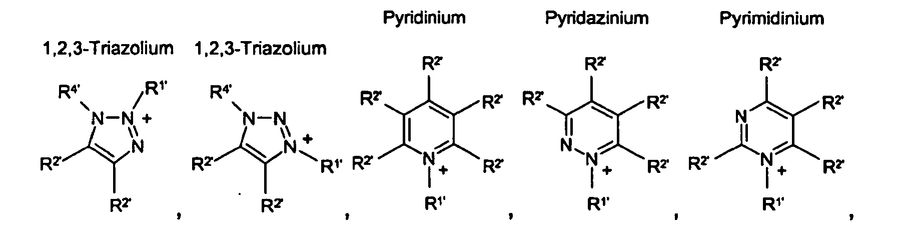 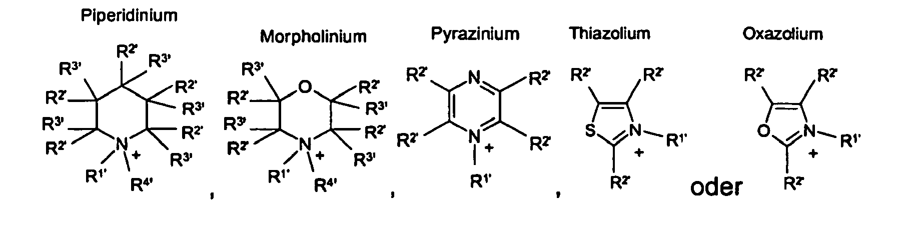 bedeutet, wobei die Substituenten
R¹' bis R⁴' jeweils unabhängig voneinander
Wasserstoff oder CN,
geradkettiges oder verzweigtes Alkyl mit 1-20 C-Atomen,
geradkettiges oder verzweigtes Alkenyl mit 2-20 C-Atomen und einer oder mehreren Doppelbindungen oder
Aryl-C₁-C₆-alkyl bedeuten,
wobei ein oder mehrere Substituenten R^{1'} bis R^{4'} teilweise oder vollständig mit Halogenen, insbesondere -F und/oder -Cl, oder teilweise mit -NO₂ oder CN substituiert sein können, wobei jedoch nicht gleichzeitig R^{1'} und R^{4'} vollständig mit Halogenen substituiert sein dürfen und
für R^{1'} bis R^{4'} = Aryl-C₁-C₆-alkyl, sowohl der Phenylring als auch die Alkylenkette teilweise oder vollständig mit Halogenen oder teilweise mit -NO₂ substituiert sein können.

12. Verfahren nach einem oder mehreren der Ansprüche 1 bis 11, **dadurch gekennzeichnet, dass** die Reaktion des Halogenids mit dem Dialkylsulfat, Alkyl-trialkylsilyl-sulfat, Alkyl-acyl-sulfat oder Alkyl-sulfonyl-sulfat ohne Lösungsmittel durchgeführt wird.

13. Eintopfverfahren zur Herstellung von Onium Alkylsulfaten, wobei die Alkylgruppe 4 bis 20 C-Atome hat, **dadurch gekennzeichnet, dass** ein Onium Halogenid mit einem symmetrisch substituierten Dialkylsulfat,
wobei die Alkylgruppe 1 bis 3 C-Atome haben kann und einem Alkohol mit 4 bis 20 C-Atomen umgesetzt wird.

14. Verwendung der Verfahren nach einem der Ansprüche 1 bis 12 oder 13 zur Reinigung von Onium Alkylsulfaten, die durch Onium-Halogenide verunreinigt sind.

15. Trialkylsilyl-octylsulfate, wobei die Alkylgruppe der Trialkylsilylgruppe jeweils unabhängig voneinander 1 bis 4 C-Atomen haben kann.

16. Verbindungen nach Anspruch 15, **dadurch gekennzeichnet, dass** die Alkylgruppen der Trialkylsilylgruppe gleich sind.

## Claims

1. Process for the preparation of onium alkylsulfates by reaction of an onium halide with a symmetrically substituted dialkyl sulfate, in which the alkyl group can have 1 to 14 C atoms, with an asymmetrically substituted dialkyl sulfate, in which one alkyl group can have 4 to 20 C atoms and the second alkyl group denotes methyl or ethyl, with an alkyl trialkylsilyl sulfate, with an alkyl acyl sulfate or with an alkyl sulfonyl sulfate, where the reaction with a dialkyl sulfate is carried out at room temperature.

2. Process according to Claim 1, **characterised in that** the reaction with a symmetrically substituted dialkyl sulfate, in which the alkyl group can have 1 to 14 C atoms, is carried out at room temperature.

3. Process according to Claim 1, **characterised in that** the reaction with an asymmetrically substituted dialkyl sulfate, in which one alkyl group can have 4 to 20 C atoms and the second alkyl group denotes methyl or ethyl, is carried out at room temperature.

4. Process according to Claim 1, **characterised in that** the reaction is carried out with an alkyl trialkylsilyl sulfate.

5. Process according to Claim 1, **characterised in that** the reaction is carried out with an alkyl acyl sulfate.

6. Process according to Claim 1, **characterised in that** the reaction is carried out with an alkyl sulfonyl sulfate.

7. Process according to one or more of Claims 1 to 6, **characterised in that** the halide is a phosphonium chloride or bromide, a guanidinium chloride or bromide, a thiouronium chloride, bromide or iodide or a chloride or bromide with a heterocyclic cation.

8. Process according to one or more of Claims 1 to 7, **characterised in that** the halide conforms to the formula (1)
[PR₄]⁺ Hal⁻ (1),
where
Hal denotes Cl or Br and
R in each case, independently of one another, denotes
H, where all substituents R cannot simultaneously be H, straight-chain or branched alkyl having 1-20 C atoms,
straight-chain or branched alkenyl having 2-20 C atoms and one or more double bonds,
where one or more R may be partially or fully substituted by halogens, in particular -F and/or -Cl, or partially by -NO₂, but where all four or three R cannot be fully substituted by halogens.

9. Process according to one or more of Claims 1 to 7, **characterised in that**
the halide conforms to the formula (2)
[C(NR¹R²)(NR³R⁴)(NR⁵R⁶)]⁺ Hal⁻ (2),
where
Hal denotes Cl or Br and
R¹ to R⁶ each, independently of one another, denote hydrogen, straight-chain or branched alkyl having 1 to 20 C atoms,
straight-chain or branched alkenyl having 2-20 C atoms and one or more double bonds,
where one or more of the substituents R¹ to R⁶ may be partially or fully substituted by halogens, in particular -F and/or -Cl, or partially by -NO₂, but where all substituents on one N atom cannot be fully substituted by halogens and
where the substituents R¹ to R⁶ may be connected to one another in pairs by a single or double bond.

10. Process according to one or more of Claims 1 to 7, **characterised in that** the halide conforms to the formula (3)
[(R¹R²N)-C(=SR⁷)(NR³R⁴)]⁺ Hal⁻ (3),
where
Hal denotes Cl, Br or I and
R¹ to R⁷ each, independently of one another, denote hydrogen, where hydrogen is excluded for R⁷, straight-chain or branched alkyl having 1 to 20 C atoms,
straight-chain or branched alkenyl having 2-20 C atoms and one or more double bonds,
where one or more of the substituents R¹ to R⁷ may be partially or fully substituted by halogens, in particular -F and/or -Cl, or partially by -NO₂, but where all substituents on one N atom cannot be fully substituted by halogens and
where the substituents R¹ to R⁷ may be connected to one another in pairs by a single or double bond.

11. Process according to one or more of Claims 1 to 7, **characterised in**
**that** the halide conforms to the formula (4)
[HetN]⁺ Hal⁻ (4),
where
Hal denotes Cl or Br and
HetN⁺ denotes a heterocyclic cation selected from the group 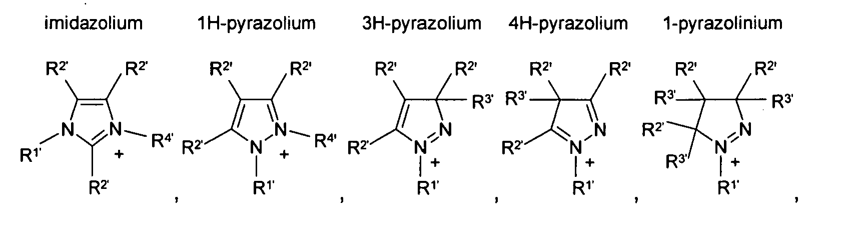 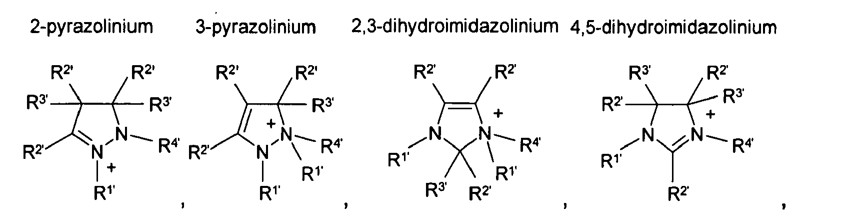 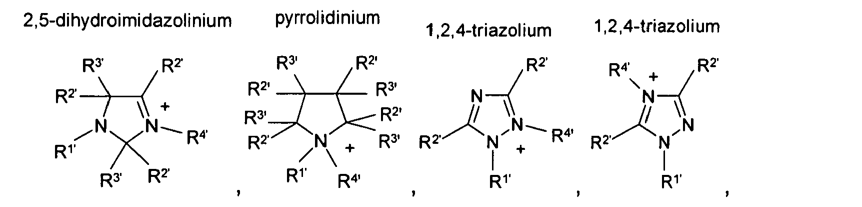 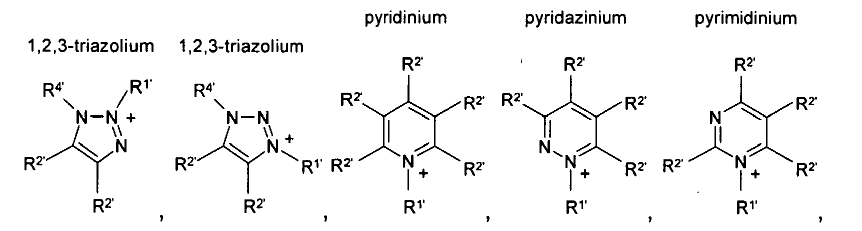 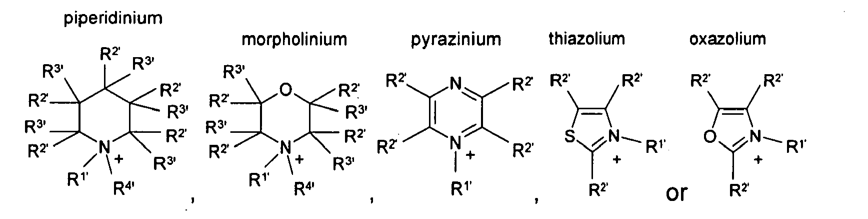 where the substituents
R¹' to R⁴' each, independently of one another, denote
hydrogen or CN,
straight-chain or branched alkyl having 1-20 C atoms,
straight-chain or branched alkenyl having 2-20 C atoms and one or more double bonds or
aryl-C₁-C₆-alkyl,
where one or more substituents R¹' to R⁴' may be partially or fully substituted by halogens, in particular -F and/or -Cl, or partially by -NO₂ or
CN, but where R^{1'} and R^{4'} cannot simultaneously be fully substituted by halogens and
for R¹' to R⁴' = aryl-C₁-C₆-alkyl, both the phenyl ring and also the alkylene chain may be partially or fully substituted by halogens or partially by -NO₂.

12. Process according to one or more of Claims 1 to 11, **characterised in that** the reaction of the halide with the dialkyl sulfate, alkyl trialkylsilyl sulfate, alkyl acyl sulfate or alkyl sulfonyl sulfate is carried out without a solvent.

13. One-pot process for the preparation of onium alkylsulfates, in which the alkyl group has 4 to 20 C atoms, **characterised in that** an onium halide is reacted with a symmetrically substituted dialkyl sulfate, in which the alkyl group can have 1 to 3 C atoms, and an alcohol having 4 to 20 C atoms.

14. Use of the processes according to one of Claims 1 to 12 or 13 for the purification of onium alkylsulfates which are contaminated by onium halides.

15. Trialkylsilyl octyl sulfates, in which the alkyl group of the trialkylsilyl group can in each case, independently of one another, have 1 to 4 C atoms.

16. Compounds according to Claim 15, **characterised in that** the alkyl groups of the trialkylsilyl group are identical.

## Revendications

1. Procédé de préparation d'alkylsulfates d'onium par la réaction d'un halogénure d'onium avec un sulfate de dialkyle symétriquement substitué, dans lequel le groupement alkyle peut avoir de 1 à 14 atomes de C, avec un sulfate de dialkyle asymétriquement substitué, dans lequel l'un des groupements alkyle peut avoir de 4 à 20 atomes de C et le deuxième groupement alkyle désigne méthyle ou éthyle, avec un sulfate d'alkyltrialkylsilyle, avec un sulfate d'alkylacyle ou avec un sulfate d'alkyl-sulfonyle, où la réaction avec un sulfate de dialkyle est réalisée à température ambiante.

2. Procédé selon la revendication 1, **caractérisé en ce que** la réaction avec un sulfate de dialkyle symétriquement substitué, dans lequel le groupement alkyle peut avoir de 1 à 14 atomes de C, est réalisée à température ambiante.

3. Procédé selon la revendication 1, **caractérisé en ce que** la réaction avec un sulfate de dialkyle asymétriquement substitué, dans lequel l'un des groupements alkyle peut avoir de 4 à 20 atomes de C et le deuxième groupement alkyle désigne méthyle ou éthyle, est réalisée à température ambiante.

4. Procédé selon la revendication 1, **caractérisé en ce que** la réaction est effectuée avec un sulfate d'alkyltrialkylsilyle.

5. Procédé selon la revendication 1, **caractérisé en ce que** la réaction est effectuée avec un sulfate d'alkylacyle.

6. Procédé selon la revendication 1, **caractérisé en ce que** la réaction est effectuée avec un sulfate d'alkylsulfonyle.

7. Procédé selon l'une ou plusieurs des revendications 1 à 6, **caractérisé en ce que** l'halogénure est un chlorure ou bromure de phosphonium, un chlorure ou bromure de guanidinium, un chlorure, bromure ou iodure de thiouronium ou un chlorure ou bromure avec un cation hétérocyclique.

8. Procédé selon l'une ou plusieurs des revendications 1 à 7, **caractérisé en ce que** l'halogénure répond à la formule (1)
[PR₄]⁺ Hal⁻ (1),
où
Hal désigne Cl ou Br et
R dans chaque cas, indépendamment l'un de l'autre, désigne
H, où tous les substituants R ne peuvent être simultanément H,
alkyle à chaîne linéaire ou ramifiée ayant 1-20 atomes de C,
alcényle à chaîne linéaire ou ramifiée ayant 2-20 atomes de C et une ou plusieurs doubles liaisons,
où un ou plusieurs R peuvent être partiellement ou totalement substitués par des halogènes, en particulier -F et/or -Cl, ou partiellement par -NO₂, mais où tous les quatre ou trois R ne peuvent être totalement substitués par des halogènes.

9. Procédé selon l'une ou plusieurs des revendications 1 à 7, **caractérisé**
**en ce que** l'halogénure répond à la formule (2)
[C(NR¹R²)(NR³R⁴)(NR⁵R⁶)]⁺ Hal⁻ (2),
où
Hal désigne Cl ou Br et
R¹ à R⁶ désignent chacun, indépendamment l'un de l'autre, hydrogène,
alkyle à chaîne linéaire ou ramifiée ayant 1 à 20 atomes de C,
alcényle à chaîne linéaire ou ramifiée ayant 2-20 atomes de C et une ou plusieurs doubles liaisons,
où un ou plusieurs parmi les substituants R¹ à R⁶ peuvent être partiellement ou totalement substitués par des halogènes, en particulier -F et/or
- CI, ou partiellement par -NO₂, mais où tous les substituants sur un atome de N ne peuvent être totalement substitués par des halogènes et
où les substituants R¹ à R⁶ peuvent être reliés les uns aux autres par paires par une liaison simple ou double.

10. Procédé selon l'une ou plusieurs des revendications 1 à 7, **caractérisé en ce que** l'halogénure répond à la formule (3)
[(R¹R²N)-C(=SR⁷)(NR³R⁴)]⁺ Hal⁻ (3),
où
Hal désigne CI, Br ou I est
R¹ à R⁷ désignent chacun, indépendamment l'un de l'autre, hydrogène, où hydrogène est exclus pour R⁷,
alkyle à chaîne linéaire ou ramifiée ayant 1 à 20 atomes de C, alcényle à chaîne linéaire ou ramifiée ayant 2-20 atomes de C et une ou plusieurs doubles liaisons,
où un ou plusieurs parmi les substituants R¹ à R⁷ peuvent être partiellement ou totalement substitués par des halogènes, en particulier -F et/or -CI, ou partiellement par -NO₂, mais où tous les substituants sur un atome de N ne peuvent être totalement substitués par des halogènes et où les substituants R¹ à R⁷ peuvent être reliés les uns aux autres par paires par une liaison simple ou double.

11. Procédé selon l'une ou plusieurs des revendications 1 à 7, **caractérisé en ce que** l'halogénure répond à la formule (4)
[HétN]⁺ Hal⁻ (4),
où
Hal désigne Cl ou Br et
HétN⁺ désigne un cation hétérocyclique choisi parmi le groupe constitué par 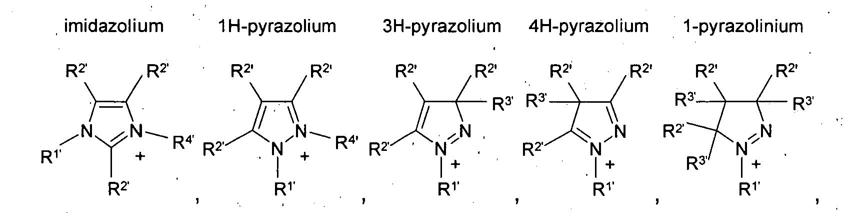 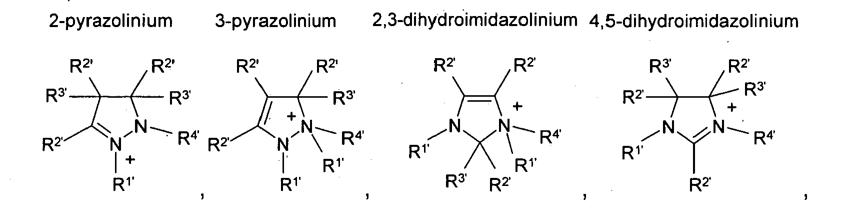 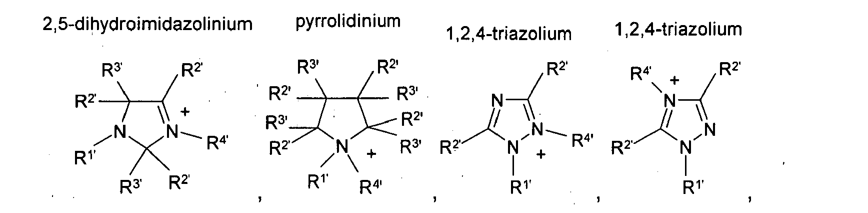 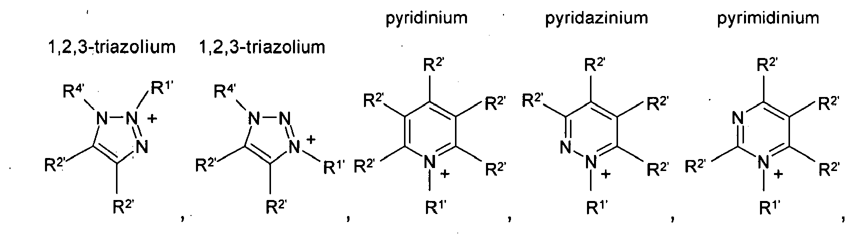 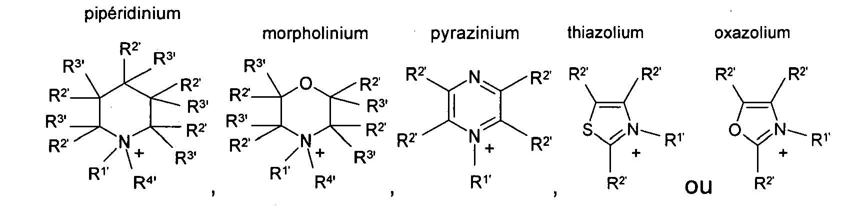 où les substituants
R¹' à R⁴' désignent chacun, indépendamment l'un de l'autre,
hydrogène ou CN,
alkyle à chaîne linéaire ou ramifiée ayant 1-20 atomes de C,
alcényle à chaîne linéaire ou ramifiée ayant 2-20 atomes de C et une ou plusieurs doubles liaisons ou
aryl-C₁-C₆-alkyle,
où un ou plusieurs substituants R¹' à R⁴' peuvent être partiellement ou totalement substitués par des halogènes, en particulier -F et/or -CI, ou partiellement par -NO₂ ou CN, mais où R^{1'} et R^{4'} ne peuvent être simultanément totalement substitués par des halogènes et
pour R¹' à R⁴' = aryl-C₁-C₆-alkyle, le cycle phényle ainsi que la chaîne allylène peuvent être partiellement ou totalement substitués par des halogènes ou partiellement par -NO₂.

12. Procédé selon l'une ou plusieurs des revendications 1 à 11, **caractérisé en ce que** la réaction de l'halogénure avec le sulfate de dialkyle, le sulfate d'alkyltrialkylsilyle, le sulfate d'alkylacyle ou le sulfate d'alkylsulfonyle est effectuée sans solvant.

13. Procédé monotope de préparation d'alkylsulfates d'onium, dans lesquels le groupement alkyle a de 4 à 20 atomes de C, **caractérisé en ce qu'**un halogénure d'onium est réagi avec un sulfate de dialkyle symétriquement substitué, où le groupement alkyle peut avoir de 1 à 3 atomes de C, et un alcool ayant de 4 à 20 atomes de C.

14. Utilisation des procédés selon l'une des revendications 1 à 12 ou 13, pour la purification d'alkylsulfates d'onium qui sont contaminés par des halogénures d'onium.

15. Sulfates de trialkylsilyloctyle, dans lesquels le groupement alkyle du groupement trialkylsilyle peut dans chaque cas, indépendamment l'un de l'autre, avoir de 1 à 4 atomes de C.

16. Composés selon la revendication 15, **caractérisés en ce que** les groupements alkyle du groupement trialkylsilyle sont identiques.
